(19) Europäisches Patentamt
European Patent Office
Office européen des brevets

(11) EP 0 948 312 B1

(12) **FASCICULE DE BREVET EUROPEEN**

(45) Date de publication et mention de la délivrance du brevet:
**21.04.2004 Bulletin 2004/17**

(21) Numéro de dépôt: **97948990.3**

(22) Date de dépôt: **03.12.1997**

(51) Int Cl.$^7$: **A61K 7/48**, A61K 7/06

(86) Numéro de dépôt international:
**PCT/FR1997/002189**

(87) Numéro de publication internationale:
**WO 1998/024408 (11.06.1998 Gazette 1998/23)**

(54) **COMPOSITIONS COSMETIQUES AQUEUSES A BASE DE SILICONES INSOLUBLES NON VOLATILS, STABILISEES PAR UN SUCCINOGLYCANE**

WÄSSERIGE KOSMETISCHE MITTEL ENTHALTEND MIT EINEM SUCCINOGLYKAN STABILISIERTE, NICHT FLÜCHTIGE UNLÖSLICHE SILIKONE

AQUEOUS COSMETIC COMPOSITIONS WITH BASE OF NON-VOLATILE INSOLUBLE SILICONS, STABILISED BY A SUCCINOGLYCAN

(84) Etats contractants désignés:
**DE ES FR GB IT**

(30) Priorité: **04.12.1996 FR 9614867**

(43) Date de publication de la demande:
**13.10.1999 Bulletin 1999/41**

(73) Titulaire: **RHODIA CHIMIE**
**92408 Courbevoie Cédex (FR)**

(72) Inventeurs:
- **GUERIN, Gilles**
  **F-95600 Eaubonne (FR)**
- **LARREY, Marie-Dominique**
  **F-75011 Paris (FR)**
- **NABAVI, Minou**
  **F-75014 Paris (FR)**
- **RICCA, Jean-Marc**
  **F-75012 Paris (FR)**

(74) Mandataire: **Delenne, Marc**
**Rhodia Services,**
**Direction de la Propriété Industrielle,**
**40, rue de la Haie-Coq**
**93306 Aubervilliers Cedex (FR)**

(56) Documents cités:

| | |
|---|---|
| **EP-A- 0 422 997** | **EP-A- 0 463 780** |
| **EP-A- 0 500 423** | **EP-A- 0 524 859** |
| **EP-A- 0 658 596** | **GB-A- 2 164 558** |

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 948 312 B1

## Description

La présente invention a pour objet des compositions cosmétiques aqueuses à effet conditionneur à base de silicone insoluble non volatil, stabilisées par un succinoglycane, leur procédé de préparation, et l'utilisation d'un succinoglycane comme agent de stabilisation des compositions cosmétiques aqueuses à effet conditionneur à base de silicone insoluble non volatil.

Le cheveu ou le corps humain se salissent au contact de la pollution atmosphérique et dans une plus large mesure par des sécrétions naturelles comme le sébum. Ce phénomène nécessite un nettoyage fréquent de la peau et des cheveux à l'aide de compositions cosmétiques. Si les propriétés détergentes de celles-ci se révèlent souvent largement satisfaisantes, des lavages fréquents et répétés ont toutefois pour effet de laisser le cheveu dans un état qui nécessite un traitement complémentaire. Différentes solutions ont été proposées pour remédier à ce problème, allant de l'usage d'agents conditionneurs inclus dans la formulation cosmétique jusqu'au développement de produits spécifiques utilisés en post traitement.

Une solution globalement satisfaisante fait appel à l'emploi de dérivés de silicone dans le shampooing comme décrit dans GB-A-849433. Une difficulté de base a été la stabilisation des particules de silicone en milieu shampooing ; une solution proposée a été l'emploi de gomme xanthane comme décrit dans US-A-4,788,006.

Cette solution nécessite néammoins l'emploi de quantités relativement importantes de gomme xanthane, de 0,4 à 5% par rapport à la formulation, pouvant provoquer des problèmes de viscosité trop importante du système, d'incompatibilité avec divers ingrédients, de stabilité à pH acide lorsque la formulation contient des hydroxyacides, ou de contribution négative à l'aspect sensoriel de la formulation, en particulier en terme de toucher cosmétique.

La présente invention a pour objet une composition cosmétique aqueuse pour le cheveu et/ou la peau, comprenant au moins un agent tensioactif, au moins un organopolysiloxane non hydrosoluble et non volatil et un agent stabilisant biopolymère, ladite composition étant caractérisée en ce que ledit agent stabilisant biopolymère est un succinoglycane.

Pour une bonne réalisation de l'invention, ladite composition peut comprendre

- de l'ordre de 1 à 60% en poids, de préférence de l'ordre de 5 à 25% en poids, d'au moins un agent tensioactif
- de l'ordre de 0,05 à 50% en poids, de préférence de l'ordre de 0,5 à 10 % en poids, d'au moins un organopolysiloxane non hydrosoluble et non volatil
- et de l'ordre de 0,01 à 5% en poids, de préférence de l'ordre de 0,05 à 0,5% en poids d'un succinoglycane.

Lesdits agents tensioactifs sont mis en oeuvre pour disperser, émulsionner, solubiliser, stabiliser divers composés utilisés notamment pour leurs propriétés émollientes ou humectantes.

Les agents tensioactifs pouvant être présents dans la composition cosmétique de l'invention, peuvent être de type anionique, non-ionique, cationique, zwitterionique ou amphotère ; on peut citer à titre d'exemples, des <u>agents tensioactifs anioniques</u> tels que

. les alkylesters sulfonates de formule R-CH($SO_3$M)-COOR', où R représente un radical alkyle en $C_8$-$C_{20}$, de préférence en $C_{10}$-$C_{16}$, R' un radical alkyle en $C_1$-$C_6$, de préférence en $C_1$-$C_3$ et M un cation alcalin (sodium, potassium, lithium), ammonium substitué ou non substitué (méthyl-, diméthyl-, triméthyl-, tetraméthylammonium, diméthylpiperidinium ...) ou dérivé d'une alcanolamine (monoéthanolamine, diéthanolamine, triéthanolamine ...). On peut citer tout particulièrement les méthyl ester sulfonates dont les radical R est en $C_{14}$-$C_{16}$ ;
. les alkylsulfates de formule $ROSO_3M$, où R représente un radical alkyle ou hydroxyalkyle en $C_{10}$-$C_{24}$, de préférence en $C_{12}$-$C_{20}$ et tout particulièrement en $C_{12}$-$C_{18}$, M représentant un atome d'hydrogène ou un cation de même définition que ci-dessus, ainsi que leurs dérivés éthoxylénés (OE) et/ou propoxylénés (OP), présentant en moyenne de 0,5 à 6 motifs, de préférence de 0,5 à 3 motifs OE et/ou OP ;
. les alkylamides sulfates de formule $RCONHR'OSO_3M$ où R représente un radical alkyle en $C_2$-$C_{22}$, de préférence en $C_6$-$C_{20}$, R' un radical alkyle en $C_2$-$C_3$, M représentant un atome d'hydrogène ou un cation de même définition que ci-dessus, ainsi que leurs dérivés éthoxylénés (OE) et/ou propoxylénés (OP), présentant en moyenne de 0,5 à 60 motifs OE et/ou OP ;
. les sels d'acides gras saturés ou insaturés en $C_8$-$C_{24}$, de préférence en $C_{14}$-$C_{20}$, les alkylbenzènesulfonates en $C_9$-$C_{20}$, les alkylsulfonates primaires ou secondaires en $C_8$-$C_{22}$, les alkylglycérol sulfonates, les acides polycarboxyliques sulfonés décrits dans GB-A-1 082 179, les sulfonates de paraffine, les N-acyl N-alkyltaurates, les alkylphosphates, les alkyliséthionates, les alkylsuccinamates les alkylsulfosuccinates, les monoesters ou diesters de sulfosuccinates, les N-acyl sarcosinates, les sulfates d'alkylglycosides, les polyéthoxycarboxylates

le cation étant un métal alcalin (sodium, potassium, lithium), un reste ammonium substitué ou non substitué (méthyl-, diméthyl-, triméthyl-, tetraméthylammonium, diméthylpiperidinium ...) ou dérivé d'une alcanolamine (monoéthanolami-

ne, diéthanolamine, triéthanolamine ...) ;

<u>agents tensio-actifs non-ioniques</u> tels que

les alkylphénols polyoxyalkylénés (polyéthoxyéthylénés, polyoxypropylénés, polyoxybutylénés) dont le substituant alkyle est en $C_6$-$C_{12}$ et contenant de 5 à 25 motifs oxyalkylènes ; à titre d'exemple, on peut citer les TRITON X-45, X-114, X-100 ou X-102 commercialisés par Rohm & Haas Cy. ;

. les glucosamides, glucamides ;
. les glycérolamides dérivés de N-alkylamines (US-A-5,223,179 et FR-A-1,585,966)
. les alcools aliphatiques en $C_8$-$C_{22}$ polyoxyalkylénés contenant de 1 à 25 motifs oxyalkylènes (oxyéthylène, oxypropylène) ; à titre d'exemple, on peut citer les TERGITOL 15-S-9, TERGITOL 24-L-6 NMW commercialisés par Union Carbide Corp., NEODOL 45-9, NEODOL 23-65, NEODOL 45-7, NEODOL 45-4 commercialisés par Shell Chemical Cy., KYRO EOB commercialisé par The Procter & Gamble Co.
. les produits résultant de la condensation de l'oxyde d'éthylène avec un composé hydrophobe résultant de la condensation de l'oxyde de propylène avec le propylène glycol, tels les PLURONIC commercialisés par BASF ;
. les oxydes d'amines tels que les oxydes d'alkyl $C_{10}$-$C_{18}$ diméthylamines, les oxydes d'alkoxy $C_8$-$C_{22}$ éthyl dihydroxy éthylamines ;
. les alkylpolyglycosides décrits dans US-A-4 565 647 et leurs dérivés polyoxyalkylénés;
. les amides d'acides gras en $C_8$-$C_{20}$
. les acides gras éthoxytés
. les amides, amines, amidoamines éthoxylées

<u>agents tensio-actifs amphotéres et zwitterioniques</u> tels que

* ceux de type bétaïne comme

  • les bétaïnes

$$R_1\ R_2\ R_3\ \overset{+}{N}R_4\ C(O)O^-$$

  • les sulfo-bétaïnes

$$R_1\ R_2\ R_3\ \overset{+}{N}R_4\ SO_3^-$$

  • les amidoalkylbétaïnes

$$R_1C(O)\ -\ NH\ R_2\overset{+}{N}\ (R_3\ R_4)\ R_5\ C(O)O^-$$

  • et les sulfo-bétaïnes

$$R_1C(O)\ -\ NH\ R_2\overset{+}{N}\ (R_3\ R_4)\ R_5\ SO_3^-$$

formules dans lesquelles les radicaux $R_1$ représente un radical alkyle ou alcényle de 10 à 24 atomes de carbone, $R_2$, $R_3$, $R_4$, et $R_5$ identiques ou différents représentent un radical alkyle ou alkylène ayant de 1 à 4 atomes de carbone.

* les alkylsultaines
* les produits de condensation d'acides gras et d'hydrolysats de proteines,
* les cocoamphoacètates et cocoamphodiacétates répondant plus généralement à la formule

$$R_1C(O)\ -\ NH\ -\ CH_2CH_2\ -\ N\ -\ CH_2CH_2\ -\ OH$$

$CH_2$ C(O)O Na

dans laquelle $R_1$ a la signification ci-dessus et représente le plus souvent des chaînes coco et lauryle.

* les alkylampho-propionates ou -dipropionates,
* les dérivés amphotères des alkylpolyamines comme l'AMPHIONIC XL® commercialisé par RHONE-POULENC, AMPHOLAC 7T/X® et AMPHOLAC 7C/X® commercialisés par BEROL NOBEL.

Parmi les organopolysiloxanes non hydrosolubles et non volatils (appelés également par la suite "silicones non hydrosolubles et non voiatils") présents dans les compositions cosmétiques faisant l'objet de l'invention, on peut citer les huiles, gommes ou résines polyalkylsiloxanes, polyarylsiloxanes, polyalkylarylsiloxanes, ou leurs dérivés fonctionallisés non hydrosolubles, ou leurs mélanges, non volatils.
Lesdits organopolysiloxanes sont considérés comme non hydrosolubles et non volatils, lorsque leur solubilité dans l'eau est inférieure à 50g/litre et leur viscosité intrinsèque d'au moins 3000 mPa.s. à 25°C.
A titre d'exemples d'organopolysiloxanes ou silicones non hydrosolubles et non volatils, on peut citer des gommes silicones comme par exemple la gomme diphenyl dimethicone commercialisée par la société Rhône-Poulenc, et de préférence les polydimethylsiloxanes présentant une viscosité au moins égale à 600000 mPa.s. à 25°C, et de façon encore plus préférentielle, ceux d'une viscosité supérieure à 2000000 mPa.s. à 25°C, tels que la Mirasil DM 500000® commercialisée par la société Rhône-Poulenc.
Les succinoglycanes sont des biopolymères de nature anioniques, dont le motif de base contient du glucose, du galactose et un reste succinyle ; ils sont décrits dans les demandes de brevet européen EP-A-351 303 et 40 445, ainsi que dans Carbohydrate Research, 73 (1979) pp.159-168, de Clarence A. Knutson ; ils peuvent être obtenus par fermentation microbienne d'un milieu comportant une source de carbone, au moyen d'un microorganisme appartenant au genre *Arthrobacter,* tel que *Arthrobacter stabilis*, en particulier la souche *Arthrobacter stabilis* NRRL-B-1973, au genre *Agrobacterium*, tels *Agrobacterium tumefaciens, Agrobacterium radiobacter* ou , *Agrobacterium rhizogenes,* au genre *Rhizobium,* en particulier *Rhizobium meliloti* et *Rhizobium trifoli*, au genre *Alcaligenes* tel *Alcaligenes faecalis*, en particulier la variété myxogenes ou au genre *Pseudomonas,* en particulier les souches *Pseudomonas sp.* NCIB 11264 et NCIB 11592 ; parmi ces succinoglycanes, on peut tout particulièrement citer les gommes rhéozan, commercialisées par Rhône-Poulenc, décrites dans la demande de brevet européen EP-A-351 303, et obtenues par fermentation d'une source carbonée au moyen de la souche *Agrobacterium tumefaciens* I-736 déposée à la collection Nationale de culture des Microorganismes (CNCM).
Au sein de la composition cosmétique faisant l'objet de l'invention, le succinoglycane peut non seulement jouer le rôle d'agent stabilisant du silicone insoluble non volatil dans le milieu aqueux, mais également celui d'agent stabilisant des particules solides comme des pigments et des nacres, des actifs cosmétiques comme des agents pelliculaires du type zinc pyrithione, ou de dispersions aqueuses comme des émulsions aqueuses d'huiles minérales ou végétales.
Selon l'invention, l'organopolysiloxane ou silicone non hydrosoluble et non volatil se trouve sous forme dispersé au sein de la composition cosmétique le renfermant. Celui-ci se présente sous forme de particules dont la taille peut être choisie en fonction de la nature de la composition cosmétique ou de la performance recherchée pour ladite composition. D'une manière générale, cette taille peut varier de 0,1 à 80 microns.
D'une manière préférentielle, cette taille est de l'ordre de 1 à 80 microns, tout particulièrement de l'ordre de 1 à 30 microns.
On entend par le terme composition ou formulation cosmétique tous les produits ou préparations cosmétiques comme celles décrites dans l'annexe I ("Illustrative list by category of cosmetic products") de la directive européenne n° 76/768/CEE du 27 juillet 1976, dite directive cosmétique.
Les compositions cosmétiques faisant l'objet de l'invention peuvent être formulées en un grand nombre de types de produits pour la peau et/ ou le cheveu , comme les mousses, les gels (coiffants notamment), les conditionneurs, les formulations pour le coiffage ou pour faciliter le peigange des cheveux, les formules de rinçage, les lotions pour les mains et le corps, les produits régulant l'hydratation de la peau, les laits de toilette, les compositions démaquillantes, les crèmes ou lotions de protection contre le soleil et le rayonnement ultra-violet, les crèmes de soins, les préparations anti acnée, les analgésiques locaux, les mascaras et bien d'autres compositions du même type.
Les compositions cosmétiques faisant l'objet de l'invention peuvent faire appel à un véhicule, ou à un mélange de plusieurs véhicules, qui sont compatibles avec une application sur le cheveu et/ou la peau. Ces véhicules peuvent être présents dans ladite composition à des concentrations comprises entre 0,5% et 99% environ, préférentiellement entre 5 et 99% environ. Le terme "compatible avec une application sur le cheveu et/ou la peau" signifie içi que le véhicule n'abime pas ou n'exerce pas d'effets négatifs sur l'aspect du cheveu et/ou de la peau ou ne crée pas d'irritation de la peau et/ou l'oeil et/ou le cuir chevelu.
Les véhicules compatibles avec les applications décrites dans cette invention comprennent par exemple ceux utilisés

dans les sprays, les mousses, les toniques, les gels, les shampoings, ou les lotions de rinçage. Le choix du véhicule approprié dépendra de la nature des ingrédients utilisés, et selon que le produit formule est censé être laissé sur la surface où il a été appliqué (par exemple sprays, mousses, loton tonique, ou gels) ou rincé après utilisation (par exemple shampoing, conditionneur, lotions de rinçage).

Les véhicules susceptibles d'être utilisés incluent un grand nombre de produits habituellement utilisés dans les compositions cosmétiques pour le cheveu et/ou la peau. Les véhicules peuvent contenir un solvant pour solubiliser ou disperser les ingrédients utilisés, avec de l'eau, des alcools en $C_1$-$C_6$, et leurs mélanges, en particulier l'eau et le méthanol, l'éthanol, l'isopropanol, et leurs mélanges. Les véhicules peuvent aussi contenir une grande variété d'autres composés comme l'acétone, les hydrocarbures (comme l'isobutane, l'hexane, le décène), les hydrocarbures halogénés (comme les fréons), le linalool, les esters (comme l'acétate d'éthyle, le phtalate de dibutyle), et des silicones volatils (en particulier les siloxanes comme le phényl pentaméthyl siloxane, le méthoxypropyl heptaméthyl cyclotétrasiloxane, le chloropropyl pentaméthyl disiloxane, l'hydropropyl pentaméthyl disiloxane, l'octaméthyl cyclotétrasiloxane, le décaméthyl cyclopenta siloxane, la cyclodiméthicone, et la diméthicone), et leurs mélanges. Lorsque ces compositions cosmétiques se présentent sous la forme de de sprays, lotions toniques, gels, ou mousses, les solvants préférentiels comprennent l'eau, l'éthanol, les dérivés volatils de silicone, et leurs mélanges. Les solvants utilisés dans ces mélanges peuvent être miscibles ou non miscibles les uns avec les autres. Les mousses et les sprays aérosol peuvent aussi utiliser n'importe quel propulseur capable de générer les produits sous forme de mousse (dans le cas des mousses) ou sous forme de sprays fins, uniformes. A titré d'exemples, on peut citer le trichlorofluorométhane, le dichlorodifluorométhane, le difluoroéthane, le diméthyléther, le propane, le n-butane, ou l'isobutane.

Dans le cas où ces compositions cosmétiques sont utilisées pour une application locale sur la peau, les véhicules doivent avoir de bonnes propriétés esthétiques, être compatibles et ne pas poser de problèmes concernant la toxicité et l'irritation.

Ces véhicules peuvent prendre un grand nombre de formes. Par exemple, les véhicules sous forme d'émulsions incluent les émulsions eau dans huile, huile dans eau, et émulsions multiples. Ces émulsions couvrent une grande plage de viscosité, de 100 à 2000000 mPa.s.. Ces émulsions peuvent aussi être délivrées sous forme de sprays en utilisant soit un dispositif de type pompe mécanique, soit sous forme d'aérosol pressurisé par l'emploi d'un gaz propulseur. Ces véhicules peuvent aussi être délivrés sous forme de mousse. On peut citer par exemple les solvants liquides anhydres, comme les huiles, les alcools et les silicones volatils, les mélanges aqueux homogènes comme les mélanges hydroalcooliques, et les versions rhéologiquement modifiées de ces deux systèmes, par exempte quand la viscosité du système a été augmentée par l'addition de gommes, résines, polymères, ou sels.

Les compositions cosmétiques faisant l'objet de l'invention peuvent en outre contenir des agents conditionneurs autres. Parmi les agents conditionneurs pouvant être utilisés dans le cadre de cette invention, on retrouve les agents conditionneurs d'origine animale, comme les hydrolysats de protéines animales, comme le sel d'ammonium de diméthyl- ou triméthyl stéarate d'hydrolysats de collagène, de soie, de kératine ; les agents conditionneurs d'origine synthétique, plus connus sous le nom polyquaternium, comme le copolymère de la N,N'-bis((diméthylamino)-3 propyl) urée et du oxy-1,1' bis(chloro-2) éthane ou polyquaternium-2, le copolymère du chlorure de diallyldiméthyl ammonium et de l'acrylamide ou polyquaternium-7 ; les dérivés cationiques de polysaccharides, comme la cellulose cocodimonium hydroxyéthyl, le guar hydroxypropyl trimonium chlorure, l'hydroxypropyl guar hydroxypropyl trimonium chlorure (JAGUAR C13S , JAGUAR C162 commercialisés par RHONE-POULENC), l'éther de poly(oxyéthanediyl-1,2) hydroxy-2 chlorure de triméthylammonium-3 propyl cellulose ou polyquaternium-10 ; les dérivés de silicones comme l'amodiméthicone, le cyclométhicone, le copolyol cétyl diméthicone, le cyclométhicone, le diméthicone copolyol, le triméthylsilyl amodiméthicone, le polyquaternium-80 ; les agents tensioactifs de type cationiques comme les halogénures de polyalkyl ammoniums par exemple le chlorure de distéaryl diméthyl ammonium.

Les agents conditionneurs sont préférentiellement choisis parmi les agents conditionneurs d'origine synthétique, en particulier les polyquaterniums -2, -7, et -10, et les dérivés cationiques de polysaccharides, comme la cellulose cocodimonium hydroxyéthyl, le guar hydroxypropyl trimonium chlorure, l'hydroxypropyl guar hydroxypropyl trimonium chlorure.

On préférera utiliser les dérivés cationiques de polysaccharides, et en particulier les dérivés de guar comme le guar hydroxypropyl trimonium chlorure et l'hydroxypropyl guar hydroxypropyl trimonium chlorure.

Les compositions cosmétiques faisant l'objet de l'invention peuvent également contenir des résines fixatives. Ces résines fixatives sont généralement présentes à des concentrations comprises entre 0.01 et 10%, préférentiellement entre 0.5 et 5%. Les résines fixatives constituants des compositions cosmétiques faisant l'objet de l'invention sont préférentiellement choisies parmi les résines suivantes :

copolymères acrylate de méthyle / acrylamide, copolymères polyvinylméthyléther / anhydride maléique, copolymères acétate de vinyle / acide crotonique, copolymères octylacrylamide / acrylate de méthyle / butylaminoéthylméthacrylate, polyvinylpyrrolidones, copolymères polyvinylpyrrolidone / méthacrytate de méthyle, copolymères polyvinylpyrrolidone / acétate de vinyle, alcools polyvinyliques, copolymères alcool polyvinylique / acide crotonique, copolymères alcool polyvinylique / anhydride maléique, hydroxypropyl celluloses, hydroxypropyl guars, polystyrène sulfonates de sodium, terpolymères polyvinylpyrrolidone / éthyl méthacrylate/ acide méthacrylique, monométhyl éthers de poly(méthylvinyl éther / acide maléique), polyvinylacétates greffés sur des troncs polyoxyéthylènes (EP-A-219 048), les copolyesters dérivés d'acide, anhydride ou d'un diester téréphtalique et/ou isophtalique et/ou sulfoisophtalique et d'un diol, tels que

- les copolymères polyesters à base de motifs ethytène téréphtalate et/ou propylène téréphtalate et polyoxyéthylène téréphtalate, (US-A-3 959 230, US-A-3 893 929, US-A-4 116 896, US-A-4 702 857, US-A-4 770 666) ;
- les oligomères polyesters sulfonés obtenus par sulfonation d'un oligomère dérivé de l'alcool allylique éthoxylé, du diméthyltéréphtalate et du 1,2 propylène diol (US-A-4 968 451)
- les copolymères polyesters à base de motifs propylène téréphtalate et polyoxyéthylène téréphtalate et terminés par des motifs éthyles, méthyles (US-A-4 711 730) ou des oligomères polyesters terminés par des groupes alkylpolyéthoxy (US-A-4 702 857) ou des groupes anioniques sulfopolyéthoxy (US-A-4 721 580), sulfoaroyles (US-A-4 877 896)
- les polyesters-polyuréthanes obtenus par réaction d'un polyester obtenu à partir d'acide adipique et/ou d'acide téréphtalique et/ou d'acide sulfoisophtalique et d'un diol, sur un prépolymère à groupements isocyanates terminaux obtenus à partir d'un polyoxyéthylène glycol et d'un diisocyanate (FR-A-2 334 698)
- les monoamines ou polyamines éthoxylées, les polymères d'amines éthoxylées (US-A-4 597 898, EP-A-11 984)
- les oligomères polyesters sulfonés obtenus par condensation de l'acide isophtalique, du sulfosuccinate de diméthyle et de diéthylène glycol (FR-A-2 236 926)
- les copolymères polyesters dérivés de diméthyltéréphtalate, d'acide isophtalique, de sulfoisophtalaîe de diméthyl et d'éthylène glycol (EP-A-540374)
- les copolymères comprenant des unités polyesters dérivés de diméthyltéréphtalate, d'acide isophtalique, de sulfoisophtalate de diméthyl et d'éthylène glycol et d'unités polyorganosiloxanes (FR-A-2 728 915).

De plus, des résines cationiques peuvent également être utilisées. Ces résines cationiques dérivent en tout ou partie de monomères cationiques comme par exemple le méthacrylate de diméthylaminoéthyle, le méthacrylate de diméthylaminoéthyle quatemarisé, le chlorure d'ammonium de diallyldiméthyle, ou leurs mélanges.

De manière préférentielle, les résines fixatives seront du type polyvinylpyrrolidone (PVP), copolymères de polyvinylpyrrolidone et de méthyl méthacrylate, copolymère de polyvinylpyrrolidone et d'acétate de vinyle (VA), copolymères polytéréphtale d'éthylène glycol / polyéthylène glycol, copolymères polytéréphtalate d'éthylène glycol / polyéthylène glycol / polyisophtalate sulfonate de sodium, et leurs mélanges.

Ces résines fixatives sont préférentiellement dispersées ou solubilisées dans le véhicule choisi.

Les compositions cosmétiques faisant l'objet de l'invention peuvent également contenir des dérivés polymériques exerçant une fonction protectrice.

Ces dérivés polymériques peuvent être présents en quantités de l'ordre de 0,01-10%, de préférence environ 0,1-5%, et tout particulièrement de l'ordre de 0.2-3% en poids, agents tels que

- les dérivés cellulosiques tels que les hydroxyéthers de cellulose, la méthylcellulose, l'éthylcellulose, l'hydroxypropyl méthylcellulose, l'hydroxybutyl méthylcellulose
- les polyvinylesters greffés sur des troncs polyalkylenes tels que les polyvinylacétates greffés sur des troncs polyoxyéthylènes (EP-A-219 048)
- les alcools polyvinyliques
- les monoamines ou polyamines éthoxylées, les polymères d'amines éthoxylées (US-A-4 597 898, EP-A-11 984)

Les performances des compositions cosmétiques faisant l'objet de l'invention peuvent aussi être améliorées par l'emploi d'agents plastifiants. L'agent plastifiant pourra constituer entre 0.1 à 20% de la formulation de préférence de 1 à 15%.Parmi les agents plastifiants particulièrement utiles, on peut citer les adipates, les phtalates, les isophtalates, les azélates, les stéarates, les silicones copolyols, les glycols, l'huile de ricin, ou leurs mélanges.

On peut aussi avantageusement ajouter à ces compositions des agents séquestrants des métaux, plus particulièrement ceux séquestrants du calcium comme les ions citrates ou des agenis émollients comme les silicones ou des huiles ou corps gras utilisés à ce propos dans l'industrie cosmétique (huiles minérales, esters d'acides gras, triglycérides, silicones, ...).

On peut également incorporer aux compositions cosmétiques faisant l'objet de l'invention des agents humectants, on peut citer le glycérol, le sorbitol, l'urée, le collagène, la gélatine, l'aloe vera, l'acide hyaluronique, et des émollients qui sont généralement choisis parmi les alkylmonoglycérides, les alkyldiglycérides, les triglycérides comme les huiles extraites des plantes et des végétaux (huiles de palme, de coprah, de graine de coton, de soja, de tournesol,

d'olive, de pépin de raisin, de sésame, d'arachide, de ricin ...) ou les huiles d'origine animale (suif, huiles de poisson ...), des dérivés de ces huiles comme les huiles hydrogénées, les dérivés de la lanoline, les huiles minérales ou les huiles paraffiniques, le perhydrosqualane, le squalène, les diols comme le 1-2-propanediol, le 1-3-butanediol, l'alcool cétylique, l'alcool stéarylique, l'alcool oléique, les polyéthylèneglycols ou polypropyléneglycols, les esters gras comme le palmitate d'isopropyl, le cocoate d'éthyl-2-hexyl, le myristyl myristate, les esters de l'acide lactique, l'acide stéarique, l'acide béhennique, l'acide isostéarique.

Pour diminuer encore l'irritation ou l'aggression du cuir chevelu, on peut aussi rajouter des polymères hydrosolubles ou hydrodispersables comme le collagène ou certains dérivés non allergisants de protéines animales ou végétales (hydrolysats de protéines de blé par exemple), des hydrocolloides naturels (gomme de guar, de caroube, de tara, ...) ou issus de procédés de fermentation et les dérivés de ces polycarbohydrates comme les celluloses modifiées (par exemple Hydroxyéthylcellulose, carboxyméthylcellulose), les dérivés du guar ou de la caroube comme leurs dérivés cationiques ou des dérivés non-ioniques (par exemple hydroxypropylguar), les dérivés anioniques (carboxyméthylguar et carboxyméthylhydroxypropylguar).

A ces composés, on peut ajouter en association des poudres ou des particules minérales comme du carbonate de calcium, des oxydés minéraux sous forme de poudre ou sous forme colloidale (particules de taille inférieure ou de l'ordre de un micromètre, parfois de quelque dizaines de nanomètres) comme du dioxyde de titane, de la silice, des sels d'aluminium utilisés généralement comme antitranspirants, du kaolin, du talc, des argiles et leurs dérivés ...

Des agents conservateurs comme les méthyl, éthyl, propyl et butyl esters de l'acide p-hydroxybenzoïque, le benzoate de sodium, le GERMABEN (nom de marque) ou tout agent chimique évitant la prolifération bactérienne ou des moisissures et utilisé traditionnellement des les compositions cosmétiques sont généralement introduits dans ces compositions à hauteur de 0,01 à 3 % en poids. La quantité de ces produits est généralement ajustée pour éviter toute prolifération de bactéries, moississures ou levures dans les compositions cosmétiques.

Alternativement à ces agents chimiques, on peut parfois utiliser des agents modifiants l'activité de l'eau et augmentant fortement la pression osmotique comme les carbohydrates ou des sels.

Pour protéger la peau et/ou les cheveux des agressions du soleil et des rayons UV, on peut ajouter à ces formulations des filtres solaires qui sont soient des composés chimiques absorbant fortement le rayonnement UV comme les composés autorisés dans la directive Européenne N° 76/768/CEE, ses annexes et les modifications ultérieures de cette directive ou des particules minérales, comme l'oxyde de zinc, le dioxyde de titane ou les oxydes de cérium sous forme de poudre ou de particules colloïdales, seuls ou en mélange. Ces poudres peuvent éventuellement être traitées en surface pour augmenter l'efficacité de leur action anti-UV ou pour faciliter leur incorporation dans les formulations cosmétiques ou pour inhiber la photoréactivité de surface.

A ces ingrédients on rajoute généralement pour augmenter l'agrément lors de l'utilisation de la composition par le consommateur, un ou des parfums, des agents colorants parmi lesquels on peut citer les produits décrits dans l'annexe IV ("List of colouring agents allowed for use in cosmetic products") de la directive européenne n° 76/768/CEE du 27 juillet 1976 dite directive cosmétique, et/ou des agents opacifiants comme des pigments. On peut aussi incorporer dans la composition des agents bactéricides ou fongicides afin d'améliorer la désinfection de la peau comme par exemple le triclosan. Des parfums, des colorants ou des pigments peuvent être ajoutés. La composition peut aussi contenir des polymères viscosants ou gélifiants, comme les polyacrylates réticulés -CARBOPOL commercialisés par GOODRICH-, les dérivés de la cellulose comme l'hydroxypropylcellulose, la carboxyméthylcellulose, les guars et leurs dérivés.... utilisés seuls ou en association, ou les mêmes composés, généralement sous la forme de polymères hydrosolubles modifiés par des groupements hydrophobes liés de manière covalente au squelette polymère comme décrit dans le brevet WO 92/16187 et/ou de l'eau pour amener le total des constituants de la formulation à 100 %.

Les compositions cosmétiques faisant l'objet de l'invention peuvent également contenir des agents dispersants polymériques en quantité de l'ordre de 0,1-7% en poids, pour contrôler la dureté en calcium et magnésium, agents tels que

. les sels hydrosolubles d'acides polycarboxyliques de masse moléculaire de l'ordre de 2000 à 100 000, obtenus par polymérisation ou copolymérisation d'acides carboxyliques éthyléniquement insaturés tels que acide acrylique, acide ou anhydride maleique, acide fumarique, acide itaconique, acide aconitique, acide mesaconique, acide citraconique, acide méthylènemalonique , et tout particulièrement les polyacrylates de masse moléculaire de l'ordre de 2 000 à 10 000 (US-A-3 308 067), les copolymères d'acide acrylique et d'anhydride maleique de masse moléculaire de l'ordre de 5 000 à 75 000 (EP-A-66 915)
. les polyéthylèneglycols de masse moléculaire de l'ordre de 1000 à 50 000

Les compositions cosmétiques ci-dessus décrites, faisant l'objet de l'invention, peuvent être préparées selon différents modes opératoires.

Elles peuvent être obtenues en mettant en oeuvre des techniques opérant à chaud en mettant en oeuvre des moyens de malaxage très cisaillants ou des techniques réalisables à température ordinaire utilisant des outils conventionnels non hautement cisaillants.

Une première technique opératoire consiste en la mise en oeuvre d'homogénéiseurs hautement cisaillants, tels que moulins colloïdaux ou UltraTurrax, et ce à chaud. Ainsi lesdites compositions peuvent être obtenues, comme décrit dans US-A-4,788,006, en réalisant les étapes suivantes :

- mélange de l'agent tensioactif ou des agents tensioactifs de la composition avec l'eau à l'aide d'un agitateur conventionnel du type turbine par exemple,
- chauffage à une température de l'ordre de 30 à 90°C, augmentation de l'agitation jusqu'à création d'un vortex et addition du succinoglycane
- addition des additifs autres que le ou les silicones non hydrosolubles et non volatils, dans les mêmes conditions d'agitation
- ajout du ou des silicones non hydrosolubles et non volatils et homogénéisation de la composition à l'aide d'un homogénéiseur, jusqu'à obtenir la taille désirée de particules de silicone(s) non hydrosoluble(s) et non volatil(s)
- et refroidissement de la composition jusqu'à température ambiante sous agitation à l'aide d'un agitateur du type turbine par exemple.

Les compositions cosmétiques ainsi obtenues présentent une distribution hétérogène de particules de silicone(s) non hydrosoluble(s) et non volatil(s) ; celle-ci peut aller de 2 à 55 microns.

Il est préférable, selon l'invention de mettre en oeuvre des techniques opératoires faisant intervenir des moyens de malaxages conventionnels non hautement cisaillants, permettant de régler la taille des particules et d'opérer à température ambiante.

Le principe de ces techniques opératoires consiste en la préparation d'une préémulsion aqueuse concentrée stabilisée dudit silicone non hydrosoluble et non volatil à l'aide d'au moins un agent tensioactif et d'une partie de la quantité totale de succinoglycane présent dans la composition cosmétique, puis en l'introduction de cette préémulsion stabilisée dans le milieu aqueux contenant la partie restante de succinoglycane et les autres additifs de la composition cosmétique.

Un premier mode opératoire, selon ce principe, consiste à préparer une préémulsion stabilisée, selon un procédé semblable à celui décrit dans EP-A-665 861.

Selon ce procédé, une préémulsion stabilisée est préparée par simple malaxage à température ambiante, d'un mélange constitué de

. 100 parties en poids de silicone(s) non hydrosoluble(s) et non volatil(s)
. de 2 à 20 parties en poids, de préférence 3 à 15 parties en poids d'eau
. de 0,5 à 10 parties en poids, de préférence 1 à 10 parties en poids d'agent(s) tensioactif(s)
. et de 0,05 à 3 parties en poids, de préférence de 0,05 à 1 partie en poids de succinoglycane

les quantités respectives des différents constituants de l'émulsion étant telles que la viscosité de la phase formée par l'eau, le' ou les agent(s) tensioactif(s) et le succinoglycane soit voisine ou supérieure à celle de la phase formée par le ou les silicone(s) non hydrosolubie(s) et non volatil(s).

Le malaxage est réalisé pendant une durée et dans des conditions de cisaillement suffisantes pour obtenir une émulsion du type "huile dans eau" de granulométrie de l'ordre de 0,1 à 5 microns, de préférence de l'ordre de 0,2 à 3 microns.

Cette préémulsion, qui présente un extrait sec en silicone non hydrosoluble et non volatil pouvant aller jusqu'à 98% en poids, est, éventuellement après dilution, introduite dans le reste de la composition cosmétique contenant la quantité restante de succinoglycane, au moins un agent tensioactif et les autres additifs éventuels de la composition ; la quantité de préémulsion introduite et des autres constituants de la composition cosmétique étant tels que ladite composition cosmétique aqueuse comprenne

- de l'ordre de 1 à 60% en poids, de préférence de l'ordre de 5 à 25% en poids, d'au moins un agent tensioactif
- de l'ordre de 0,05 à 50% en poids, de préférence de l'ordre de 0,5 à 10% en poids, d'au moins un organopolysiloxane non hydrosoluble et non volatil
- et de l'ordre de 0,01 à 5% en poids, de préférence de l'ordre de 0,05 à 0,5% en poids d'un succinoglycane.

Un deuxième mode opératoire, selon le même principe, mettant en oeuvre des moyens de malaxages traditionnels non hautement cisaillants du type pâle raclante, considéré comme préférentiel et constituant un deuxième objet ce l'invention, consiste à préparer d'abord une première préémulsion aqueuse constituée de

- de 70 à 96 parties en poids d'au moins silicone non hydrosoluble et non volatil,
- de 0,01 à 20 parties en poids d'au moins un agent tensioactif,
- le complément à 100 parties en poids étant réalisé par de l'eau, la quantité d'eau présente étant d'au moins 3 parties en poids,

et à y introduire de 0,01 à 5 parties en poids de succinoglycane sous forme d'une solution aqueuse pour obtenir une préémulsion stabilisée dont la concentration en silicone non hydrosoluble et non volatil est de l'ordre de 20 à 80% en poids, de préférence de l'ordre de 50 à 70% en poids.

La première préémulsion peut être préparée, et ce de façon non limitative, de la manière suivante.

Le silicone non hydrosoluble et non volatil est introduit dans un réacteur conventionnel équipé d'une agitation faiblement cisaillante de type pâle raclante. L'addition de l'eau et du ou des tensioactifs est réalisée à température ambiante, à une vitesse qui n'est pas critique, l'emploi d'un prémélange eau/tensioactifs pouvant être réalisé pour des raisons de commodités. L'agitation est maintenue pendant un temps nécessaire à l'obtention de la granulométrie souhaitée, ce temps variant de quelques minutes à quelques dizaines de minutes en fonction de l'équipement utilisé.

Les conditions de malaxage (durée et cisaillement) sont telles que la première préémulsion, ainsi que la préémulsion stabilisée sont du type "huile dans eau" et présentent une de granulométrie de l'ordre de 1 à 75 microns, de préférence de l'ordre de 1 à 30 microns.

La préémulsion stabilisée, qui présente un extrait sec en silicone non hydrosoluble et non volatil pouvant aller jusqu'à 80% en poids, est diluée si nécessaire jusqu'à un extrait sec en silicone non hydrosoluble et non volatil ne dépassant pas 70% en poids, de préférence de l'ordre de 50 à 70% en poids, et introduite dans le reste de la composition cosmétique contenant la quantité restante de succinoglycane, un ou des agent(s) tensioactif(s) et les autres additifs éventuels de la composition, la quantité de préémulsion stabilisée introduite, la quantité restante de succinogtycane, la quantité d'agent(s) tensioaetif(s) et des autres additifs éventuels de la composition cosmétique étant telles que ladite composition cosmétique aqueuse comprenne

- de l'ordre de 1 à 60% en poids, de préférence de l'ordre de 5 à 25% en poids, d'au moins un agent tensioactif
- de l'ordre de 0,05 à 50% en poids, de préférence de l'ordre de 0,5 à 10% en poids, d'au moins un organopolysiloxane non hydrosoluble et non volatil
- et de l'ordre de 0,01 à 5% en poids, de préférence de l'ordre de 0,05 à 0,5% en poids d'un succinoglycane.

Le ou les agent(s) tensioactif(s) mis en oeuvre pour réaliser la mise en préémulsion stabilisée du ou des silicone(s) non hydrosoluble(s) et non volatil(s) selon les deux modes opératoires décrits, peuvent être du même type que ceux déjà mentionnés ci-dessus. D'une manière préférentielle, ceux-ci sont des alcools aliphatiques en $C_8$-$C_{22}$ polyoxyalkylénés contenant de 1 à 25 motifs oxyalkylènes (oxyéthylène, oxypropylène), et de manière encore plus préférentielle des alcools de type laurique éthoxylés comprenant de 5 à 12 motifs oxyalkylénés.

Le choix du ou des agents tensioactifs est de préférence effectué non seulement sur la base de leurs propriétés émulsifiantes mais aussi sur la base de la charge nette résiduelle de la particule de silicone non hydrosoluble et non volatil, mesurée par le potentiel zéta, cette charge nette pouvant être ajustée de façon à optimiser l'affinité de la dispersion pour une surface donnée (cheveu ou peau).

La taille des particules est en outre contrôlée par la nature du ou des tensioactifs, et par la position dans le diagramme de phase ternaire eau - silicone non hydrosoluble et non volatil - tensioactif (s), c'est-à-dire par la concentration en agent tensio-actif dans ladite préémulsion stabilisée par rapport à la quantité d'eau présente.

A titre de règle générale, dans le cas d'une huile polydimethyl siloxane de viscosité supérieure à 50000 mPa.s. à 25°C et constituant au moins 80% de la dispersion finale, les petites tailles seront obtenues par l'emploi de grandes quantités de tensioactifs par rapport à l'eau, les grandes tailles par l'emploi de faibles quantités de tensioactifs par rapport à l'eau.

Un troisième objet de l'invention consiste en l'utilisation d'au moins un succinoglycane comme agent de stabilisation des compositions cosmétiques aqueuses comprenant au moins un silicone non hydrosoluble et non volatil et au moins un agent tensioactif.

Pour une bonne réalisation de l'invention, ledit succinoglycane est utilisé en quantité telle que ladite composition cosmétique aqueuse comprenne

- de l'ordre de 1 à 60% en poids, de préférence de l'ordre de 5 à 25% en poids, d'au moins un agent tensioactif
- de l'ordre de 0,05 à 50% en poids, de préférence de l'ordre de 0,5 à 10 % en poids, d'au moins un organopolysiloxane non hydrosoluble et non volatil
- et de l'ordre de 0,01 à 5% en poids, de préférence de l'ordre de 0,05 à 0,5% en poids de succinoglycane.

La nature des succinoglycanes, du ou des agent(s) tensioactif(s), du ou des silicone(s) non hydrosoluble(s) et non volatil(s) et des autres additifs eventuels présents dans lesdites compositions a déjà été donnée ci-dessus.

Un dernier objet de l'invention consiste en un procédé de stabilisation des compositions cosmétiques aqueuses comprenant au moins un silicone non hydrosoluble et non volatil et au moins un agent tensioactif, par addition d'au moins un succinoglycane auxdites compositions.

Pour une bonne réalisation de l'invention, ledit succinoglycane est additionné en quantité telle que ladite composition cosmétique aqueuse stabilisée comprenne

- de l'ordre de 1 à 60% en poids, de préférence de l'ordre de 5 à 25% en poids, d'au moins un agent tensioactif
- de l'ordre de 0,05 à 50% en poids, de préférence de l'ordre de 0,5 à 10 % en poids, d'au moins un organopolysiloxane non hydrosoluble et non volatil
- et de l'ordre de 0,01 à 5% en poids, de préférence de l'ordre de 0,05 à 0,5% en poids de succinoglycane.

La nature des succinoglycanes, du ou des agent(s) tensioactif(s), du ou des silicone(s) non hydrosoluble(s) et non volatil(s) et des autres additifs éventuels présents dans lesdites compositions a déjà été donnée ci-dessus.
Les exemples suivants sont donnés à titre illustratif.

**EXEMPLE 1**

préparation d'une émulsion concentrée calibrée (2µm) de polydiméthylsiloxane

Dans un réacteur inox d'un volume de 2 litres muni d'un agitateur de type pale raclante, on introduit 950g de Mirasil DM 500000 (polydiméthylsiloxane commercialisé par Rhône-Poulenc). Sous agitation, on ajoute 100 grammes d'une solution de laureth-8 dans l'eau distillée contenant 85g de matière active (lauryl alcool contenant 8 motifs oxyéthylénés). Le mélange résiduel est agité pendant cinq minutes et on obtient alors une émulsion monodispersée dont la taille moyenne des particules est de 2 microns.

**EXEMPLE 2**

préparation d'une émulsion concentrée calibrée (20µm) de polydiméthylsiloxane

Dans un réacteur inox d'un volume de 2 litres muni d'un agitateur de type pale raclante, on introduit 950g de Mirasil DM 500000 (polydiméthylsiloxane commercialisé par Rhône-Poulenc). Sous agitation, on ajoute 100 grammes d'une solution de laureth-8 dans l'eau distillée contenant 45g de matière active. Le mélange résiduel est agité pendant cinq minutes et on obtient alors une émulsion monodispersée dont la taille moyenne des particules est de 20 microns.

**EXEMPLE 3**

stabilisation d'une émulsion concentrée calibrée (2µm ou 20µm) de polydiméthylsiloxane

A 0,553 kg de l'émulsion concentrée préparée dans l'exemple 1 ou 2, on ajoute sous agitation 0,447 kg une solution de Rhéozan (commercialisé par Rhône-Poulenc) dans l'eau contenant 10g de matière active. On obtient ainsi une émulsion stable D ou H contenant 50% de Mirasil DM 500000 et 0,1% de Rhéozan.
La stabilité de cette émulsion après 24 heures à 20°C et 40°C, est comparée à celle de l'émulsion A ou E ne contenant pas d'agent stabilisant, à celle de l'émulsion B ou F stabilisée par 0,1% de gomme xanthane et à celle de l'émulsion C ou G stabilisée par 0,3% de gomme xanthane.
Les résultats obtenus sont donnés dans le tableau suivant

| **composition (% en poids)**<br>DM 500000 | **EMULSION** | | | | | | | |
|---|---|---|---|---|---|---|---|---|
| | **A** | **B** | **C** | **D** | **E** | **F** | **G** | **H** |
| | 50 | 50 | 50 | 50 | 50 | 50 | 50 | 50 |
| gomme xanthane | 0 | 0,1 | 0,3 | 0 | 0 | 0,1 | 0,3 | 0 |
| rhéozan | 0 | 0 | 0 | 0,1 | 0 | 0 | 0 | 0,1 |
| **taille microns** | 2 | 2 | 2 | 2 | 20 | 20 | 20 | 20 |
| **stabilité**<br>20°C/24 hrs<br>45°C/24 hrs | crémage<br>crémage | crémage<br>crémage | stable<br>stable | stable<br>stable | crémage<br>crémage | crémage<br>crémage | stable<br>crémage | stable<br>stable |

On constate que le rhéozan présente des propriétés stabilisantes supérieures à celles de la gomme xanthane et que

son emploi permet de diminuer considérablement la concentration en biopolymère.

**EXEMPLE 4**

stabilité en formule shampooing

Une formule type de shampoing est préparée par mélange dans l'eau distillée de sodium laureth-2 sulfate (pour obtenir 9% en poids de matière active - Empicol ESB/3M commercialisé par Allbright & Wilson) et de sodium coco amphoacétate (pour obtenir 3% en poids de matière active - Miranol Ultra C32 commercialisé par Rhône-Poulenc). La viscosité est ajustée par ajout de chlorure de sodium. Différentes quantités de gomme xanthane ou de rhéozan sont est ensuite ajoutées. On obtient ainsi des bases shampoings.
Les émulsions concentrées D, H, B, F, C et G sont directement incorporées dans cette base shampooing pour obtenir une formulation shampoing présentant une concentration de 2% en silicones. On obtient des formulations D', H', B', F', C' et G'.

La stabilité des formulations D' et H' contenant 0.1% de rhéozan, après 24 heures à 20°C et 40°C, est comparée à celle des émulsions A' et E' ne contenant pas d'agent stabilisant, à celle des émulsions B' et F' stabilisées par 0,1% de gomme xanthane et à celle des émulsion C' et G' stabilisée par 0,3% de gomme xanthane.

Les résultats obtenus sont donnés dans le tableau suivant

| **composition (% en poids)** émulsion concentrée incorporée | **FORMULATION** | | | | | | | |
|---|---|---|---|---|---|---|---|---|
| | **A'** | **B'** | **C'** | **D'** | **E'** | **F'** | **G'** | **H'** |
| | A | B | C | D | E | F | G | H |
| concentration en silicone | 2 | 2 | 2 | 2 | 2 | 2 | 2 | 2 |
| gomme xanthane totale | 0 | 0,1 | 0,3 | 0 | 0 | 0,1 | 0,3 | 0 |
| rhéozan total | 0 | 0 | 0 | 0,1 | 0 | 0 | 0 | 0,1 |
| **taille microns** | 2 | 2 | 2 | 2 | 20 | 20 | 20 | 20 |
| **stabilité** 20°C/24 hrs | crémage | crémage | stable | stable | crémage | crémage | stable | stable |
| 45°C/24 hrs | crémage | crémage | stable | stable | crémage | crémage | crémage | stable |

On constate que le rhéozan présente des propriétés stabilisantes supérieures à celles de la gomme xanthane en formulation shampoing et que son emploi permet de diminuer considérablement la concentration en biopolymère. La réduction de cette concentration permet de diminuer la contribution négative des biopolymères à certaines caractéristiques cosmétiques de la formulation.

**EXEMPLE 5**

shampoing 2-en-1

On prépare comme ci-après indiqué, un shampoing 2-en-1 de composition suivante:

| INGREDIENTS | % en poids |
|---|---|
| Sodium Laureth-2 Sulfate (27% de matière active) | 30.0 |
| Miranol Ultra C32 (sodium coco ampho acetate) | 6 |
| **émulsion concentrée H** | 2.0 |
| Lauramide de DEA (Lauramide de diéthanolamine) | 2.0 |
| Glycol Distearate | 2.0 |
| PEG-150 Distearate<br>(distéarate de polyethylène glycol à 150 motifs oxyéthylènes) | 1.5 |
| **Rheozan total** | 0.1 |
| Germaben II (conservateur)<br>(Propyiene glycol +)Diazolinidyl urée + Methylparaben + Propylparaben) | 0,2 |
| parfum | 0,05 |
| Polysorbate 20<br>(ester de sorbitan à 20 motifs oxyéthylènes) | 0,2 |
| acide citrique | pour obtenir un pH =7 |
| eau distillée | qsp 100 |

On disperse le Rheozan dans l'eau sous agitation. On ajoute ensuite le conservateur et maintient l'agitation jusqu'à dissolution complète. Le Sodium Laureth Sulfate et le Miranol Ultra C32 sont ensuite ajoutés par portions sous agitation modérée. L'agitation est poursuivie jusqu'à l'obtention d'un milieu homogène. On introduit le Lauramide de DEA, on chauffe à 75°C ± 5°C et ajoute le distéarate d'éthylène glycol et le distéarate de PEG-150. Lorsque le milieu devient homogène, on refroidit à température ambiante sous agitation. On ajoute graduellement l'émulsion concentrée H et le mélange Polysorbate 20 / parfum. Le milieu est agité jusqu'à complète dispersion. Le pH est ensuite ajusté à 7 avec de l'acide citrique.
On obtient un liquide perlescent présentant une viscosité de 3500 ± 500 mPa.s. (Brookfield, spindle n°4, 10 tours/minute, 25°C), et un pH 7.
Les performances de cette formulation sont validées par un panel d'analyse sensorielle et sont jugées satisfaisantes sur l'aspect pouvoir conditionneur.

**EXEMPLE 6**

shampoing conditionneur

On prépare comme ci-après indiqué, un shampoing conditionneur de composition suivante:

| ingrédients | % en poids |
|---|---|
| JAGUAR C13S eau distillée | 0.5 41 |
| sodium laureth sulfate (solution à 27% d'extrait sec) eau distillée | 40 13.5 |
| cocoamido propyl betaine (solution à 30% d'extrait sec) | 4 |

(suite)

| ingrédients | % en poids |
|---|---|
| **émulsion concentrée H** | 1 |
| **Rheozan total** | 0.1 |
| conservateur | 0.1 |
| parfum | 0,05 |
| hydroxyde de sodium (solution à 25%) | pour obtenir un pH = 6,5 |

Le Jaguar C13S est dissous sous agitation dans l'eau distillée. Le laureth sulfate de sodium, préalablement dissous dans l'eau, y est ensuite introduit. La cocoamido propyl betaine est ajoutée et le milieu obtenu est agité jusqu'à homogénéité. Le rhéozan est alors incorporé. L'émulsion concentrée H est ensuite dispersée lentement dans le milieu. Le conservateur et le parfum sont alors ajoutés. La viscosité est ajustée ainsi que le pH par ajout d'hydroxyde de sodium. Les performances de cette formulation sont validées par un panel d'analyse sensorielle et sont jugées satisfaisantes sur l'aspect peignabilité sur cheveu mouillé.

**EXEMPLE 7**

<u>Savon</u>

On prépare un pain de savon de composition suivante :

| ingrédients | % en poids |
|---|---|
| Geropon AS-200 (cocoiséthionate de sodium) | 64,82 |
| acide stéarique | 11,24 |
| base savon | 8,54 |
| Geropon T-77 (N méthyl-N oleyltaurate de sodium) | 3,80 |
| iséthionate de sodium (55%) | 4,32 |
| butylhydroxytoluène | 0,01 |
| EDTA (sel de sodium de l'éthylène diamine tetraacétate) | 0,05 |
| émulsion concentrée H | 3,30 |
| dioxyde de titane | 0,30 |
| huile parfumante | 0,50 |
| eau | 3,12 |

**Revendications**

1. Composition cosmétique aqueuse pour le cheveu et/ou la peau, comprenant au moins un agent tensioactif, au moins un organopolysiloxane non hydrosoluble et non volatil et un agent stabilisant biopolymère, ladite composition étant **caractérisée en ce que** ledit agent stabilisant biopolymère est un succinoglycane.

2. Composition selon la revendication 1), **caractérisée en ce qu'**elle comprend :

   - de l'ordre de 1 à 60% en poids, de préférence de l'ordre de 5 à 25% en poids, d'au moins un agent tensioactif
   - de l'ordre de 0,05 à 50% en poids, de préférence de l'ordre de 0,5 à 10 % en poids, d'au moins un organopolysiloxane non hydrosoluble et non volatil
   - et de l'ordre de 0,01 à 5% en poids, de préférence de l'ordre de 0,05 à 0,5% en poids d'un succinoglycane.

3. Composition selon la revendication 1) ou 2), **caractérisée en ce que** ledit organopolysiloxane non hydrosoluble

et non volatil se présente sous forme de particules dispersées de taille de l'ordre de 0,1 à 80 microns, de préférence de l'ordre de 1 à 80 microns, tout particulièrement de l'ordre de 1 à 30 microns.

**4.** Procédé de préparation de la composition cosmétique aqueuse faisant l'objet de l'une quelconque des revendications 1) à 3), caractérisé. en l'obtention d'une préémulsion aqueuse concentrée stabilisée dudit silicone non hydrosoluble et non volatil à l'aide d'au moins un agent tensioactif et d'une partie de la quantité totale de succinoglycane présent dans la composition cosmétique, puis en l'introduction de cette préémulsion stabilisée dans le milieu aqueux comprenant la partie restante de succinoglycane.

**5.** Procédé selon la revendication 4), **caractérisé en ce que** ladite préémulsion aqueuse concentrée stabilisée dudit silicone non hydrosoluble et non volatil est obtenue par préparation d'une première préémulsion aqueuse constituée de

- de 70 à 96 parties en poids d'au moins un silicone non hydrosoluble et non volatil,
- de 0,01 à 20 parties en poids d'au moins un agent tensioactif,
- le complément à 100 parties en poids étant réalisé par de l'eau, la quantité d'eau présente étant d'au moins 3 parties en poids,

et introduction dans ladite première préémulsion, de 0,01 à 5 parties en poids de succinoglycane sous forme d'une solution aqueuse pour obtenir une préémulsion stabilisée dont la concentration en silicone non hydrosoluble et non volatil est de l'ordre de 20 à 80% en poids, de préférence de l'ordre de 50 à 70% en poids.

**6.** Procédé selon la revendication 5), **caractérisé en ce que** la première préémulsion, ainsi que la préémulsion stabilisée sont du type "huile dans eau" et présentent une de granulométrie de l'ordre de 1 à 75 microns, de préférence de l'ordre de 1 à 30 microns.

**7.** Procédé selon la revendication 5) ou 6), **caractérisé en ce que** ladite préémulsion concentrée stabilisée est diluée si nécessaire jusqu'à un extrait sec en silicone non hydrosoluble et non volatil ne dépassant pas 70% en poids, de préférence de l'ordre de 50 à 70% en poids, et introduite dans le reste de la composition cosmétique contenant la quantité restante de succinoglycane, un ou des agent(s) tensioactif(s). et les autres additifs éventuels de la composition, la quantité de préémulsion stabilisée introduite, la quantité restante de succinoglycane, la quantité d'agent(s) tensioactif(s) et des autres additifs éventuels de la composition cosmétique étant telles que ladite composition cosmétique aqueuse comprenne

- de l'ordre de 1 à 60% en poids, de préférence de l'ordre de 5 à 25% en poids, d'au moins un agent tensioactif
- de l'ordre de 0,05 à 50% en poids, de préférence de l'ordre de 0,5 à 10% en poids, d'au moins un organopolysiloxane non hydrosoluble et non volatil
- et de l'ordre de 0,01 à 5% en poids, de préférence de l'ordre de 0,05 à 0,5% en poids d'un succinoglycane.

**8.** Utilisation d'au moins un succinoglycane comme agent de stabilisation des compositions cosmétiques aqueuses comprenant au moins un silicone non hydrosoluble et non volatil et au moins un agent tensioactif.

**9.** Utilisation selon la revendication 8), **caractérisée en ce que** ledit succinoglycane est utilisé en quantité telle que ladite composition cosmétique aqueuse comprenne

- de l'ordre de 1 à 60% en poids, de préférence de l'ordre de 5 à 25% en poids, d'au moins un agent tensioactif
- de l'ordre de 0,05 à 50% en poids, de préférence de l'ordre de 0,5 à 10 % en poids, d'au moins un organopolysiloxane non hydrosoluble et non volatil
- et de l'ordre de 0,01 à 5% en poids, de préférence de l'ordre de 0,05 à 0,5% en poids de succinoglycane.

**10.** Procédé de stabilisation des compositions cosmétiques aqueuses comprenant au moins un silicone non hydrosoluble et non volatil et au moins un agent tensioactif, par addition d'au moins un succinoglycane auxdites compositions.

**11.** Procédé selon la revendication 10) **caractérisé en ce que** ledit succinoglycane est additionné en quantité telle que ladite composition cosmétique aqueuse stabilisée comprenne

- de l'ordre de 1 à 60% en poids, de préférence de l'ordre de 5 à 25% en poids, d'au moins un agent tensioactif

- de l'ordre de 0,05 à 50% en poids, de préférence de l'ordre de 0,5 à 10 % en poids, d'au moins un organopolysiloxane non hydrosoluble et non volatil
- et de l'ordre de 0.01 à 5% en poids, de préférence de l'ordre de 0,05 à 0,5% en poids de succinoglycane.

**Claims**

**1.** Aqueous cosmetic composition for the hair and/or the skin, comprising at least one surfactant, at least one water-insoluble, non-volatile organopolysiloxane and a biopolymeric stabilizer, the said composition being **characterized in that** the said biopolymeric stabilizer is a succinoglycan.

**2.** Composition according to Claim 1, **characterized in that** it comprises:

- from about 1 to 60% by weight, preferably from about 5 to 25% by weight, of at least one surfactant
- from about 0.05 to 50% by weight, preferably from about 0.5 to 10% by weight, of at least one water-insoluble, non-volatile organopolysiloxane, and
- from about 0.01 to 5% by weight, preferably from about 0.05 to 0.5% by weight, of a succinoglycan.

**3.** Composition according to Claim 1 or 2, **characterized in that** the said water-insoluble, non-volatile organopolysiloxane is in the form of dispersed particles with a size of about 0.1 to 80 microns, preferably of about 1 to 80 microns, most particularly of about 1 to 30 microns.

**4.** Process for the preparation of the aqueous cosmetic composition forming the subject of any one of Claims 1 to 3, **characterized by** the production of a stabilized, concentrated, aqueous preemulsion of the said water-insoluble, non-volatile silicone using at least one surfactant and some of the total amount of succinoglycan present in the cosmetic composition, followed by the introduction of this stabilized preemulsion into the aqueous medium comprising the remaining succinoglycan.

**5.** Process according to Claim 4, **characterized in that** the said stabilized concentrated aqueous preemulsion of the said water-insoluble, non-volatile silicone is obtained by preparation of a first aqueous preemulsion consisting of

- from 70 to 96 parts by weight of at least one water-insoluble, non-volatile silicone,
- from 0.01 to 20 parts by weight of at least one surfactant,
- the remainder to 100 parts by weight being made up of water, the amount of water present being at least 3 parts by weight, and introduction into the said first preemulsion of from 0.01 to 5 parts by weight of succinoglycan in the form of an aqueous solution in order to obtain a stabilized preemulsion whose water insoluble, non-volatile silicone concentration is from about 20 to 80% by weight, preferably from about 50 to 70% by weight.

**6.** Process according to Claim 5, **characterized in that** the first preemulsion and the stabilized preemulsion are of the "oil-in-water" type and have a particle size of about 1 to 75 microns, preferably of about 1 to 30 microns.

**7.** Process according to Claim 5 or 6, **characterized in that** the said stabilized concentrated preemulsion is diluted, if necessary, to a solids content of water-insoluble, non-volatile silicone not exceeding 70% by weight, preferably of about 50 to 70% by weight, and is introduced into the remainder of the cosmetic composition containing the remaining amount of succinoglycan, one or more surfactant(s) and the other optional additives of the composition, the amount of stabilized preemulsion introduced, the remaining amount of succinoglycan, the amount of surfactant (s) and of the other optional additives of the cosmetic composition being such that the said aqueous cosmetic composition comprises

- from about 1 to 60% by weight, preferably from about 5 to 25% by weight, of at least one surfactant
- from about 0.05 to 50% by weight, preferably from about 0.5 to 10% by weight, of at least one water-insoluble, non-volatile organopolysiloxane, and
- from about 0.01 to 5% by weight, preferably from about 0.05 to 0.5% by weight, of a succinoglycan.

**8.** Use of at least one succinoglycan as a stabilizer for aqueous cosmetic compositions comprising at least one water-insoluble, non-volatile silicone and at least one surfactant.

**9.** Use according to Claim 8, **characterized in that** the said succinoglycan is used in an amount such that the said

aqueous cosmetic composition comprises

- from about 1 to 60% by weight, preferably from about 5 to 25% by weight, of at least one surfactant
- from about 0.05 to 50% by weight, preferably from about 0.5 to 10% by weight, of at least one water-insoluble, non-volatile organopolysiloxane, and
- from about 0.01 to 5% by weight, preferably from about 0.05 to 0.5% by weight, of succinoglycan.

**10.** Process for stabilizing aqueous cosmetic compositions comprising at least one water-insoluble, non-volatile silicone and at least one surfactant, by addition of at least one succinoglycan to the said compositions.

**11.** Process according to Claim 10, **characterized in that** the said succinoglycan is added in an amount such that the said stabilized aqueous cosmetic composition comprises

- from about 1 to 60% by weight, preferably from about 5 to 25% by weight, of at least one surfactant
- from about 0.05 to 50% by weight, preferably from about 0.5 to 10% by weight, of at least one water-insoluble, non-volatile organopolysiloxane, and
- from about 0.01 to 5% by weight, preferably from about 0.05 to 0.5% by weight, of succinoglycan.

## Patentansprüche

**1.** Wäßrige kosmetische Zusammensetzung für Haar und/oder Haut, die mindestens ein Tensid (oberflächenaktive Substanz), mindestens ein nichtwasserlösliches und nichtflüchtiges Organopolysiloxan und einen biopolymeren Stabilisator aufweist, **dadurch gekennzeichnet, daß** der biopolymere Stabilisator ein Succinoglykan ist.

**2.** Zusammensetzung nach Anspruch 1, **dadurch gekennzeichnet, daß** sie umfaßt:

— etwa 1 bis 60 Gew.-%, vorzugsweise etwa 5 bis 25 Gew.-%, mindestens eines Tensids,

— etwa 0,05 bis 50 Gew.-%, vorzugsweise etwa 0,5 bis 10 Gew.-%, mindestens eines nichtwasserlöslichen und nichtflüchtigen Organopolysiloxans und

— etwa 0,01 bis 5 Gew.-%, vorzugsweise etwa 0,05 bis 0,5 Gew.-%, eines Succinoglykans.

**3.** Zusammensetzung nach Anspruch 1 oder 2, **dadurch gekennzeichnet, daß** das nichtwasserlösliche und nichtflüchtige Organopolysiloxan in Form dispergierter Partikel (Teilchen) mit einer Größe von etwa 0,1 bis 80 µm (Mikron), vorzugsweise etwa 1 bis 80 µm, besonders bevorzugt etwa 1 bis 30 µm, vorliegt.

**4.** Verfahren zur Herstellung der wäßrigen kosmetischen Zusammensetzung nach einem der Ansprüche 1 bis 3, **gekennzeichnet durch** den Erhalt einer stabilisierten, konzentrierten wäßrigen Präemulsion des nichtwasserlöslichen und nichtflüchtigen Silikons mit Hilfe mindestens eines Tensids und eines Teils der Gesamtmenge des in der kosmetischen Zusammensetzung vorhandenen Succinoglykans, dann Zugabe dieser stabilisierten Präemulsion in das wäßrige Medium, das den restlichen Teil des Succinoglykans enthält.

**5.** Verfahren nach Anspruch 4, **dadurch gekennzeichnet, daß** die stabilisierte, konzentrierte wäßrige Präemulsion des nichtwasserlöslichen und nichtflüchtigen Silikons erhalten wird durch Herstellung einer ersten wäßrigen Präemulsion, die

— 70 bis 96 Gewichtsteile mindestens eines nichtwasserlöslichen und nichtflüchtigen Silikons,

— 0,01 bis 20 Gewichtsteile mindestens eines Tensids,

— wobei das Ganze mit Wasser auf 100 Gewichtsteile zu ergänzen ist, wobei die Menge an vorhandenem Wasser mindestens 3 Gewichtsteile beträgt,

enthält, und
Zugabe von 0,01 bis 5 Gewichtsteilen Succinoglykan in Form einer wäßrigen Lösung zu der ersten Präemulsion, um eine stabilisierte Präemulsion zu erhalten, deren Konzentration an nichtwasserlöslichem und nichtflüchtigem

Silikon etwa 20 bis 80 Gew.-%, vorzugsweise etwa 50 bis 70 Gew.-%, beträgt.

**6.** Verfahren nach Anspruch 5, **dadurch gekennzeichnet, daß** die erste Präemulsion sowie die stabilisierte Präemulsion vom "Öl-in-Wasser"-Typ sind und eine Granulometrie bzw. Teilchengröße von etwa 1 bis 75 µm, vorzugsweise etwa 1 bis 30 µm, aufweisen.

**7.** Verfahren nach Anspruch 5 oder 6, **dadurch gekennzeichnet, daß** die stabilisierte konzentrierte Präemulsion gegebenenfalls auf einen Trockenextrakt an nichtwasserlöslichem und nichtflüchtigem Silikon von nicht mehr als 70 Gew.-%, vorzugsweise etwa 50 bis 70 Gew.-%, verdünnt wird und zu dem Rest der kosmetischen Zusammensetzung, der die restliche Menge an Succinoglykan, Tensid(en) und gegebenenfalls weiteren Additiven der Zusammensetzung enthält, zugegeben wird, wobei die Menge an zugegebener stabilisierter Präemulsion, die restliche Menge an Succinoglykan und die Menge an Tensid(en) und an gegebenenfalls weiteren Additiven der kosmetischen Zusammensetzung derart sind, daß die wäßrige kosmetische Zusammensetzung

— etwa 1 bis 60 Gew.-%, vorzugsweise etwa 5 bis 25 Gew.-%, mindestens eines Tensids,

— etwa 0,05 bis 50 Gew.-%, vorzugsweise etwa 0,5 bis 10 Gew.-%, mindestens eines nichtwasserlöslichen und nichtflüchtigen Organopolysiloxans und

— etwa 0,01 bis 5 Gew.-%, vorzugsweise etwa 0,05 bis 0,5 Gew.-%, Succinoglykan

enthält.

**8.** Verwendung mindestens eines Succinoglykans als Stabilisierungsreagenz für wäßrige kosmetische Zusammensetzungen, die mindestens ein nichtwasserlösliches und nichtflüchtiges Silikon und mindestens ein Tensid enthalten.

**9.** Verwendung nach Anspruch 8, **dadurch gekennzeichnet, daß** das Succinoglykan in solchen Mengen verwendet wird, daß die wäßrige kosmetische Zusammensetzung

— etwa 1 bis 60 Gew.-%, vorzugsweise etwa 5 bis 25 Gew.-%, mindestens eines Tensids,

— etwa 0,05 bis 50 Gew.-%, vorzugsweise etwa 0,5 bis 10 Gew.-%, mindestens eines nichtwasserlöslichen und nichtflüchtigen Organopolysiloxans und

— etwa 0,01 bis 5 Gew.-%, vorzugsweise etwa 0,05 bis 0,5 Gew.-%, Succinoglykan

enthält.

**10.** Verfahren zur Stabilisierung von wäßrigen kosmetischen Zusammensetzungen, die mindestens ein nichtwasserlösliches und nichtflüchtiges Silikon und mindestens ein Tensid enthalten, durch Zugabe mindestens eines Succinoglykans zu diesen Zusammensetzungen.

**11.** Verfahren nach Anspruch 10, **dadurch gekennzeichnet, daß** das Succinoglykan in solchen Mengen zugegeben wird, daß die stabilisierte, wäßrige kosmetische Zusammensetzung

— etwa 1 bis 60 Gew.-%, vorzugsweise etwa 5 bis 25 Gew.-%, mindestens eines Tensids,

— etwa 0,05 bis 50 Gew.-%, vorzugsweise etwa 0,5 bis 10 Gew.-%, mindestens eines nichtwasserlöslichen und nichtflüchtigen Organopolysiloxans und

— etwa 0,01 bis 5 Gew.-%, vorzugsweise etwa 0,05 bis 0,5 Gew.-%, Succinoglykan

enthält.